# EUROPÄISCHE PATENTSCHRIFT

(11) **EP 2 810 935 B1**
(45) Veröffentlichungstag und Bekanntmachung des Hinweises auf die Patenterteilung: **25.05.2016**
(21) Anmeldenummer: 13170255.7
(22) Anmeldetag: 03.06.2013
(51) Int. Cl.: C07C 305/06, C07C 303/24, C11D 1/14

(54) **Sulfatierte Ester von Oligohydroxycarbonsäuren und deren Verwendung**
Sulfated esters of oligohydroxy carboxylic acids and their use
Esters sulfatés d'acides oligohydroxycarboniques et leur utilisation

(43) Veröffentlichungstag der Anmeldung: 10.12.2014
(73) Patentinhaber: BASF SE, 67056 Ludwigshafen am Rhein (DE)
(72) Erfinder: Behler, Ansgar, 46240 Bottrop (DE); Clasen, Frank, 40589 Hilden (DE)
(74) Vertreter: BASF IP Association

(56) Entgegenhaltungen:
- EP-A1- 0 530 866

## Beschreibung

### HINTERGRUND DER ERFINDUNG

Die vorliegende Erfindung betrifft sulfatierte Ester von Oligohydroxycarbonsäuren, kosmetische und pharmazeutische Mittel, die diese Ester enthalten sowie die Verwendung dieser Ester als anionische Tenside.

### STAND DER TECHNIK

Anionische Tenside gehören zu den am weitesten verbreiteten grenzflächenaktiven Verbindungen und werden außer in Wasch- und Reinigungsmittel auch auf dem Gebiet der Kosmetik vielfältig eingesetzt. Übliche anionische Tenside, wie sie vor allem in der Kosmetik eingesetzt werden, sind die Salze von Alkylethersulfaten (Alkylpolyethersulfate, Fettalkoholpolyglycolethersulfate, verkürzt auch Ethersulfate). Sie zeichnen sich durch starkes Schaumvermögen, hohe Reinigungskraft, geringe Härte- und Fettempfindlichkeit aus und finden vielfach Verwendung zur Herstellung von kosmetischen Produkten wie beispielsweise Haarshampoos, Schaum- oder Duschbädern, aber auch in Handgeschirrspülmitteln.

Für viele aktuelle Anwendungen werden an anionische Tenside außer einer guten grenzflächenaktiven Wirkung weitere Anforderungen gestellt. Insbesondere in der Kosmetik ist eine hohe dermatologische Verträglichkeit erforderlich. Des Weiteren ist in der Regel eine ausreichende Wasserlöslichkeit, eine gute Verträglichkeit mit möglichst vielen der in der Kosmetik eingesetzten Wirk- und Hilfsstoffen, ein gutes Schaumbildungsvermögen und ein gutes Rheologieverhalten erwünscht. Des Weiteren besteht ein Bedarf an anionischen Tensiden, die zumindest teilweise aus biogenen Quellen und speziell auch nachwachsenden Rohstoffen hergestellt werden können. Weiterhin besteht auch ein Bedarf an Tensiden, die keine alkoxilierten Gruppen aufweisen und die somit insbesondere den Einsatz von Ethylenoxid zu ihrer Herstellung überflüssig machen.

Die EP 0 530 866 A1 beschreibt sulfatierte Ester von Alkancarbonsäuren und deren Verwendung als oberflächenaktive Verbindungen, die bei Hautkontakt hydrolysiert werden und eine Wirkkomponente (z. B. einen Hydroxycarbonsäureester) freisetzen.

Die DE 40 03 096 A1 beschreibt sulfatierte Hydroxycarbonsäureester und deren Verwendung als oberflächenaktive Substanzen. Das auf Seite 4 dieses Dokuments beschriebene Beispiel A zur Herstellung von Milchsäurelaurylester ist keine ausführbare technische Lehre zur Herstellung von Laurylestern von Milchsäureoligomeren. So gelingt es bereits nicht, die Veresterung unter Verwendung von Toluol als Schleppmittel, welches mit dem bei der Reaktion freiwerdenden Wasser ein Azeotrop mit einem Siedepunkt von etwa 80 bis 90 °C bildet, unter Verwendung eines Wasserabscheiders auf die angegebenen 225 °C aufzuheizen.

Die DE 43 40 042 A1 beschreibt die Verwendung von Sulfat-Tensiden, wie sie in der DE 40 03 096 A1 beschrieben werden, als oberflächenaktive Substanzen in Reinigungs- und Spülmitteln zum Reinigen und Spülen harter Oberflächen.

Der vorliegenden Erfindung liegt die Aufgabe zugrunde, neue Verbindungen bereitzustellen, die sich vorteilhaft als grenzflächenaktive Verbindungen für diverse Anwendungen eignen. Sie sollen speziell geeignet sein, ein komplexes Anforderungsspektrum abzudecken, wie es eingangs beschrieben ist. Insbesondere soll es möglich sein, tensidhaltige Formulierungen bereitzustellen, die über anwendungstechnische Eigenschaften verfügen, die zumindest vergleichbar sind mit aus dem Stand der Technik bekannten anionischen Tensiden auf Basis von petrochemischen Komponenten und/oder auf Basis von Alkylenoxiden.

Überraschenderweise wurde nun gefunden, dass diese Aufgabe durch sulfatierte Ester von Oligohydroxycarbonsäuren gelöst wird.

### ZUSAMMENFASSUNG DER ERFINDUNG

Ein erster Gegenstand der Erfindung sind Verbindungen der allgemeinen Formel (I) 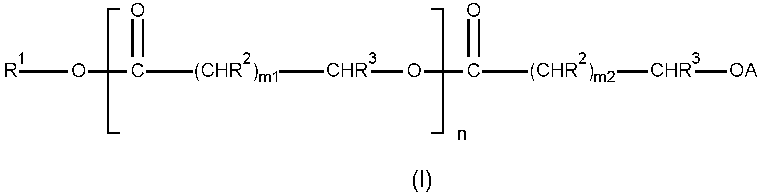 worin
R¹ für Wasserstoff oder einen linearen oder verzweigten aliphatischen Kohlenwasserstoffrest mit 1 bis 30 Kohlenstoffatomen und 0, 1, 2 oder 3 Doppelbindungen steht,
die Reste R² unabhängig voneinander ausgewählt sind unter Wasserstoff, Methyl, Ethyl, -OA, -COOR⁴, -CH₂-OA und -CH₂-COOR⁴, wobei die Reste R⁴ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
die Reste R³ unabhängig voneinander ausgewählt sind unter Wasserstoff, Methyl, Ethyl, -OA, -COOR⁵, -CH₂-OA und -CH₂-COOR⁵, wobei die Reste R⁵ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
A für H oder -SO₃B steht, worin B für Wasserstoff oder ein Kationenäquivalent steht,
n im Mittel für einen Wert von wenigstens 0,1 steht,
m1 und m2 unabhängig voneinander für 0 oder 1 stehen,
mit der Maßgabe, dass wenigstens einer der Reste A für -SO₃B steht, und
mit der Maßgabe, dass wenigstens einer der Reste R¹, R⁴ oder R⁵ für einen linearen oder verzweigten aliphatischen Kohlenwasserstoffrest mit 1 bis 30 Kohlenstoffatomen und 0, 1, 2 oder 3 Doppelbindungen steht.

Eine bevorzugte Ausführungsform sind sulfatierte Ester von Oligolactaten. Danach sind die Verbindungen der allgemeinen Formel (I) ausgewählt unter Verbindungen (I) nach einem der vorhergehenden Ansprüche, die ausgewählt sind unter Verbindungen der Formel (I.1) 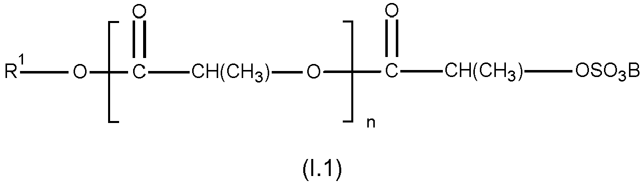 worin
- R¹: für Wasserstoff oder einen linearen oder verzweigten Kohlenwasserstoffrest mit 6 bis 30 Kohlenstoffatomen und 0, 1, 2 oder 3 Doppelbindungen steht,
- B: für Wasserstoff oder ein Kationenäquivalent steht, und
- n: im Mittel für einen Wert von wenigstens 0,1 steht.

Ein weiterer Gegenstand der Erfindung ist ein Verfahren zur Herstellung von Verbindungen der allgemeinen Formel (I). Gegenstand der Erfindung sind auch die nach diesem Verfahren erhältlichen Verbindungen der allgemeinen Formel (I).

Ein weiterer Gegenstand der Erfindung ist eine kosmetische oder pharmazeutische Zusammensetzung, die wenigstens eine Verbindung der allgemeinen Formel (I), wie zuvor und im Folgenden definiert, enthält.

Ein weiterer Gegenstand der Erfindung ist die Verwendung von Verbindungen der allgemeinen Formel (I), wie zuvor und im Folgenden definiert, als oberflächenaktive Substanzen, speziell als anionisches Tensid für Wasch- und Reinigungsmittel, kosmetische Mittel, pharmazeutische Mittel.

### BESCHREIBUNG DER ERFINDUNG

Die Verbindungen der allgemeinen Formel (I) können in Form von Mischungen oder als Reinverbindungen vorliegen. Für die erfindungsgemäßen Verwendungen eignen sich in der Regel Mischungen von Verbindungen der allgemeinen Formel (I), wie sie z. B. durch das im Folgenden beschriebene Herstellungsverfahren erhältlich sind. Die einzelnen Komponenten dieser Mischungen können sich beispielsweise hinsichtlich des Oligomerisierungsgrads n unterscheiden. Werden zur Herstellung der Verbindungen der allgemeinen Formel (I) Hydroxycarbonsäuren eingesetzt, die mehr als eine Carboxylgruppe und/oder mehr als eine alkoholische OH-Gruppe aufweisen, so kann es sich bei den einzelnen Komponenten dieser Mischungen auch um Strukturisomere aus der Veresterungsreaktion zu ihrer Herstellung handeln. Selbstverständlich ist es auch möglich, die nach dem erfindungsgemäßen Verfahren erhältlichen Reaktionsgemische nach üblichen Trennverfahren, z. B. destillativ oder chromatographisch, aufzutrennen.

Der mittlere Oligomerisierungsgrad ergibt sich für die erfindungsgemäßen Verbindungen der allgemeinen Formel (I) und der allgemeinen Formel (I.1) durch Addition von 1 zum Wert der Variablen n.

Im Rahmen der Erfindung bezeichnet Kationäquivalent ein einwertiges Kation oder den einwertigen Ladungsanteil eines mehrwertigen Kations.

Wenn B für ein Kationäquivalent steht, so ist dieses vorzugsweise ausgewählt unter Alkalimetallkationen, NH₄⁺ und Kationen der Formel HNE¹E²E³⁺, wobei E¹, E² und E³ unabhängig voneinander ausgewählt sind unter Wasserstoff, linearem und verzweigtem C₁-C₆-Alkyl und linearem und verzweigtem C₁-C₄-Hydroxyalkyl, mit der Maßgabe, dass einer der Reste E¹, E² und E³ von Wasserstoff verschieden ist. Bevorzugt ist das Kationäquivalent ausgewählt unter Na⁺, K⁺, NH₄⁺, Mg²⁺/2, HN(CH₃)₃⁺, HN(C₂H₅)₃⁺, HN(C₂H₄OH)₃⁺, H₂N(C₂H₄OH)₂⁺, etc.

Geeignete lineare oder verzweigte aliphatische Kohlenwasserstoffreste mit 1 bis 30 Kohlenstoffatomen und 0, 1, 2 oder 3 Doppelbindungen sind die entsprechenden C₁-C₃₀-Alkylreste, C₁-C₃₀-Alkenylreste, C₁-C₃₀-Alkadienylreste und C₁-C₃₀-Alkatrienylreste.

Bevorzugt steht wenigstens einer der Reste R¹, R⁴ oder R⁵ für einen lineare oder verzweigte aliphatischen Kohlenwasserstoffreste mit 1 bis 30 Kohlenstoffatomen und 0, 1, 2 oder 3 Doppelbindungen. Insbesondere sind R¹, R⁴ und R⁵ unabhängig voneinander ausgewählt unter Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2 Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, Isotridecyl, Isostearyl, Oleyl, Linoleyl, Linolenyl, etc.

In einer bevorzugten Ausführung steht wenigstens einer der Reste R¹, R⁴ oder R⁵ für einen linearen oder verzweigten aliphatischen Kohlenwasserstoffrest mit 6 bis 30 Kohlenstoffatomen und 0, 1, 2 oder 3 Doppelbindungen. Besonders bevorzugt sind R¹, R⁴ und R⁵ unabhängig voneinander ausgewählt unter n-Hexyl, 2-Ethylhexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, Isotridecyl, Isostearyl, Oleyl, Linoleyl, Linolenyl, und Kombinationen davon.

Die Reste R¹, R⁴ und R⁵ können sich von reinen Alkoholen oder von Alkoholgemischen ableiten. Bevorzugt handelt es sich um großtechnische verfügbare Alkohole oder Alko-holgemische. In einer bevorzugten Ausführungsform sind R¹, R⁴ und R⁵ dann unabhängig voneinander ausgewählt unter überwiegend linearen Alkyl-, Alkenyl-, Alkadienyl- und Alkatrienylresten, wie sie in natürlichen oder synthetischen Fettsäuren und den entsprechenden Fettalkoholen vorkommen.

In einer weiteren bevorzugten Ausführungsform sind R¹, R⁴ und R⁵ unabhängig voneinander abgeleitet von Fettalkoholen, die auf technischen Alkoholmischungen basieren. Dazu zählen z. B. die bei der Hydrierung von technischen Methylestern auf Basis von Fetten und Ölen anfallenden Alkoholgemische. Dazu zählen weiterhin die bei der Hydrierung von Aldehyden aus der Oxosynthese anfallenden Alkoholgemische (Oxoalkohole) oder die bei der Dimerisierung von ungesättigten Fettalkoholen anfallenden Alkoholgemische.

Bevorzugt leitet sich wenigstens einer der Reste R¹, R⁴ und R⁵ von linearen gesättigten Alkoholen mit 8 bis 18 Kohlenstoffatomen ab.

Besonders bevorzugt leitet sich wenigstens einer der Reste R¹, R⁴ und R⁵ von einem Gemisch linearer gesättigter C₁₂-/C₁₄-Alkohole ab.

Des Weiteren bevorzugt leitet sich wenigstens einer der Reste R¹, R⁴ und R⁵ von einem C₁₆-/C₁₈-Fettalkoholgemisch ab. Gemische aus Cetyl (Hexadecyl) und Stearyl (Octadecyl) werden auch als Cetearyl bezeichnet.

Bevorzugt haben in den Verbindungen (I) die Variablen m1 und m2 die gleiche Bedeutung.

Bei den Verbindungen der allgemeinen Formel (I) handelt es sich um Ester von Oligohydroxycarbonsäuren. Diese können sich von üblichen Hydroxycarbonsäuren ableiten, wie Milchsäure, Glycolsäure, Äpfelsäure, Weinsäure, Tartronsäure und Mischungen davon. Bevorzugt sind die Verbindungen (I) abgeleitet von Milchsäure, Glycolsäure, Äpfelsäure, Weinsäure oder Mischungen davon. Besonders bevorzugt sind die Verbindungen (I) abgeleitet von Milchsäure.

In den Verbindungen der allgemeinen Formel (I) steht n vorzugsweise für einen Wert von 0,1 bis 100, besonders bevorzugt von 0,15 bis 50, insbesondere von 0,2 bis 20.

In den Verbindungen der allgemeinen Formel (I.1) steht n vorzugsweise für einen Wert von 0,1 bis 100, besonders bevorzugt von 0,15 bis 50, insbesondere von 0,2 bis 20.

Ein weiterer Gegenstand der Erfindung ist ein Verfahren zur Herstellung von Verbindungen der allgemeinen Formel (I), bei dem man
a) wenigstens eine Hydroxycarbonsäure der allgemeinen Formel (I.A) 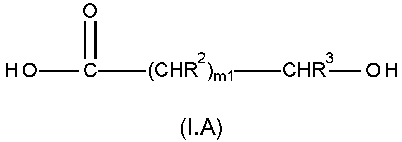 worin R², R³ und m1 wie in einem der Ansprüche 1 bis 8 definiert sind, in einer Veresterungsreaktion umsetzt, wobei die Veresterung in Gegenwart wenigstens eines Alkohols R¹-OH erfolgt, wobei R¹ wie in einem der Ansprüche 1 bis 8 definiert ist oder das Produkt der Veresterung der Hydroxycarbonsäure(n) (I.A) anschließend mit wenigstens einem Alkohol R¹-OH umgesetzt wird,
b) das Reaktionsprodukt aus Schritt a) mit einem Sulfatierungsmittel umsetzt, und
c) gegebenenfalls das Reaktionsprodukt aus Schritt b) mit einer Base zumindest teilweise neutralisiert.

Bezüglich geeigneter und bevorzugter Definitionen von R¹, R², R³, R⁴, R⁵ und m1 wird auf die vorherigen Ausführungen zu diesen Resten und Variablen in vollem Umfang Bezug genommen.

### Schritt a)

Die Veresterungsreaktion in Schritt a) kann mehrstufig erfolgen, wobei zuerst wenigstens eine Hydroxycarbonsäure (I.A) einer Veresterung unter Oligomerisierung unterzogen wird und anschließend das erhaltene Reaktionsgemisch, gegebenenfalls nach einer Auftrennung und/oder Reinigung, in einer weiteren Veresterung mit wenigstens einem Alkohol R¹-OH umgesetzt wird.

Vorzugsweise erfolgt die Veresterungsreaktion zur Herstellung von Verbindungen der allgemeinen Formel (I) im Sinne einer Eintopfreaktion, bei der man wenigstens eine Hydroxycarbonsäure der allgemeinen Formel (I.A) in Gegenwart wenigstens eines Alkohols R¹-OH einer Veresterung unterzieht.

Die Veresterungsreaktion kann nach allgemein bekannten Verfahren erfolgen, wobei die Entfernung des gebildeten Reaktionswassers z. B. durch wasserentziehende Mittel, extraktiv oder destillativ erfolgen kann.

Vorzugsweise wird das gebildete Reaktionswasser destillativ entfernt. In einer speziellen Ausführungsform kann das gebildete Reaktionswasser azeotrop entfernt werden. Die Umsetzung erfolgt dabei in Gegenwart eines Lösungsmittels, das mit Wasser ein azeotropes Gemisch bildet. Geeignete Lösungs- und Schleppmittel sind aliphatische und aromatische Kohlenwasserstoffe, z. B. Alkane, wie n-Hexan und n-Heptan, Cycloalkane, wie Cyclohexan und Methylcyclohexan, Aromaten, wie Benzol, Toluol und Xylolisomere und sogenannte Spezialbenzine, welche Siedepunkte zwischen 70 und 140 °C aufweisen. Besonders bevorzugte Schleppmittel sind Cyclohexan, Methylcyclohexan und Toluol. Geeignete Apparaturen zur azeotropen Destillation unter Abscheidung des Reaktionswassers und Rückführung des Lösungsmittels in das Reaktionsgefäß sind dem Fachmann bekannt. Das eingesetzte Lösungsmittel kann nach der Veresterung durch übliche Methoden, wie z. B. durch Destillation, gegebenenfalls unter vermindertem Druck, aus dem Reaktionsgemisch entfernt werden.

Wird zur Veresterung ein Alkohol R¹-OH mit ausreichend hohem Siedepunkt eingesetzt, z. B. ein gesättigter oder ein- oder mehrfach ungesättigter Fettalkohol mit wenigstens 6 Kohlenstoffatomen, so kann bei der destillativen Entfernung des Reaktionswassers auf den Einsatz eines Schleppmittels verzichtet werden.

Die Veresterungstemperatur liegt im Allgemeinen in einem Bereich von etwa 50 bis 250 °C, besonders bevorzugt von 70 bis 200 °C.

Die Veresterung erfolgt vorzugsweise bei Normaldruck oder vermindertem Druck. Zur destillativen Abtrennung des Reaktionswassers ist es vorteilhaft, die Veresterung bei vermindertem Druck durchzuführen. Bevorzugt liegt der Druck bei der Veresterung in einem Bereich von 1 mbar bis 1,1 bar, insbesondere 5 mbar bis 1 bar, speziell 10 mbar bis 900 mbar. Dies gilt sowohl für die zuvor beschriebene einstufige als auch die zweistufige Variante der Veresterungsreaktion.

Die Veresterung kann autokatalytisch oder in Gegenwart eines Katalysators erfolgen. Geeignete Katalysatoren sind starke Säuren, wie z. B. Schwefelsäure, wasserfreier Chlorwasserstoff, Sulfonsäuren, z. B. Toluolsulfonsäure und Methansulfonsäure, und saure Ionenaustauscher. Im erfindungsgemäßen Verfahren wird vorzugsweise Schwefelsäure und p-Toluolsulfonsäure als Katalysator eingesetzt. Die Menge des Veresterungskatalysators liegt dabei im Allgemeinen in einem Bereich von etwa 0,1 bis 5 Gew.-%, bezogen auf die Gesamtmenge an zu veresternden Komponenten.

Bevorzugt erfolgt die Veresterungsreaktion ohne Zusatz eines externen Lösungsmittels. Dies gilt sowohl für die zuvor beschriebene einstufige als auch die zweistufige Variante der Veresterungsreaktion. Es ist jedoch alternativ möglich, die erfindungsgemäße Umsetzung in Gegenwart eines unter den Reaktionsbedingungen inerten, organischen Lösemittels oder eines Lösungsmittelgemischs durchzuführen. Bevorzugt sind aprotische Lösemittel. Die eingesetzten Lösungsmittel weisen vorzugsweise einen Siedepunkt von wenigstens 120 °C, insbesondere von wenigstens 140 °C auf. Dazu zählen z. B. Alkylenglycoldialkylether wie Ethylenglykoldimethylether, Ethylenglykoldiethylether, etc. Ebenfalls geeignet sind z. B. Cyclohexanon (Sdp.: 155 °C), N-Methylpyrrolidon (Sdp.: 204 °C), Sulfolan (Sdp.: 285 °C), Nitrobenzol (Sdp.: 210 °C), Xylol (Sdp.: 140 °C).

Die Einstellung des gewünschten mittleren Oligomerisierungsgrads (p = 1 + n) gelingt in fachüblicher Weise.

So kann bei der zuvor beschriebenen zweistufigen Variante der Veresterungsreaktion zunächst eine oligomere Hydroxycarbonsäure mit dem gewünschten mittleren Oligomerisierungsgrad (p = 1 + n) hergestellt werden. Dazu wird das Reaktionsgemisch unter wasserentziehenden Bedingungen für einen Zeitraum bei der Reaktionstemperatur belassen, der ausreicht, um den angestrebten mittleren Oligomerisierungsgrad zu erreichen. Vorzugsweise beträgt die Reaktionsdauer etwa 0,5 bis etwa 24 Stunden, insbesondere etwa 1 bis etwa 20 Stunden. Anschließend wird die so erhaltene oligomere Hydroxycarbonsäure mit wenigstens einem Alkohol R¹-OH zum Endprodukt umgesetzt. Das molare Verhältnis von Alkoholkomponente R¹-OH zu oligomerer Hydroxycarbonsäure beträgt bei dieser Variante etwa 1:1.

Bei der zuvor beschriebenen einstufigen Variante der Veresterungsreaktion wird wenigstens eine Hydroxycarbonsäure der allgemeinen Formel (I.A) in Gegenwart wenigstens eines Alkohols R¹-OH einer Veresterung unterzogen. Nach dieser Variante gelingt die Einstellung des gewünschten mittleren Oligomerisierungsgrads n beispielsweise über das molare Verhältnis von Alkoholkomponente R¹-OH zu Hydroxycarbonsäure (I.A). Dieses beträgt bei dieser Variante bevorzugt 1 : 1,01 bis 1 : 200, besonders bevorzugt 1 : 1,1 bis 1 : 100, insbesondere 1 : 1,15 bis 1 : 50, speziell 1 : 1,2 bis 1 : 20. Auch nach dieser Variante wird das Reaktionsgemisch unter wasserentziehenden Bedingungen für einen Zeitraum bei der Reaktionstemperatur belassen, der ausreicht, um Verbindungen der allgemeinen Formel (I) mit dem angestrebten mittleren Oligomerisierungsgrad zu erreichen. Vorzugsweise beträgt die Reaktionsdauer etwa 0,5 bis etwa 24 Stunden, insbesondere etwa 1 bis etwa 20 Stunden.

### Schritt b)

Sulfatierungsreaktionen von Tensidalkoholen sind dem Fachmann bekannt und z. B. in der WO 93/24453, DE 40 03 096 A1 sowie in "Ullmann's Encyclopedia of Industrial Chemistry", 5. Auflage Bd. A25 (1994), Seiten 779-783 und in den dort angegebenen Literaturverweisen beschrieben.

Prinzipiell können zur Sulfatierung des Reaktionsprodukts aus Schritt a) Schwefeltrioxid, Schwefeltrioxid-Komplexe, Lösungen von Schwefeltrioxid in Schwefelsäure ("Oleum"), konzentrierte Schwefelsäure, Chlorsulfonsäure, Sulfurylchlorid oder auch Amidosulfosäure eingesetzt werden.

Bevorzugt umfasst das in Schritt b) eingesetzte Sulfatierungsmittel SO₃ oder besteht aus SO₃. Bevorzugt wird gasförmiges Schwefeltrioxid im Gemisch mit einem unter den Reaktionsbedingungen der Sulfatierung inerten Gas eingesetzt. Bevorzugte inerte Gase sind Stickstoff oder Luft. Dabei wird das Schwefeltrioxid mit Luft oder Stickstoff verdünnt und vorzugsweise in Form eines Gasgemisches mit ca. 1 bis 10, insbesondere 3 bis 6 Vol.-%, Schwefeltrioxid eingesetzt.

Die Sulfierung mit SO₃ kann kontinuierlich oder diskontinuierlich, insbesondere jedoch in Reaktoren durchgeführt werden, die nach dem Fallfilmprinzip arbeiten.

Bevorzugt wird die Reaktion in Abwesenheit von Lösungsmitteln durchgeführt. Es können jedoch auch für die Sulfatierung von Olefinen, Aromaten, Alkoholen und dergleichen übliche Lösungsmittel, wie z. B. Orthoameisensäureester, Dimethylformamid, 1,2-Dichlorethan oder Tetrahydrofuran, eingesetzt werden.

Die Sulfatierung mit SO₃ wird vorzugsweise mit einem molaren Verhältnis von Hydroxycarbonsäureester zu SO₃ von 1 : 0,9 bis 1 : 2,4 durchgeführt. Besonders bevorzugt ist dabei ein Bereich von 1 : 1,0 bis 1 : 1,3.

Die Sulfatierung mit SO₃ wird beispielsweise bei Temperaturen von 10 bis 98 °C durchgeführt. Um einerseits eine ausreichend niedrige Viskosität der Einsatzstoffe zu gewährleisten und andererseits eine zu starke thermische Belastung während der Reaktion zu vermeiden, empfiehlt es sich, die Sulfierung bei einer Temperatur im Bereich von 20 bis 90 °C durchzuführen.

Wird Chlorsulfonsäure als sulfatierendes Reagenz eingesetzt, so wird die entsprechende Alkoholkomponente vorzugsweise in einer Rührapparatur unter inerten Bedingungen vorgelegt und die Chlorsulfonsäure zugeführt. Das Molverhältnis zwischen Alkoholkomponente und Chlorsulfonsäure liegt vorzugsweise bei 0,5 : 1 bis 1 : 0,5, besonders bevorzugt liegt das Verhältnis bei 0,75 : 1 bis 1 : 0,75. Ganz besonders bevorzugt liegt das Molverhältnis von Alkoholkomponente zu Chlorsulfonsäure bei etwa 1 : 1.

Wird Schwefelsäure zur Veresterung eingesetzt, so verwendet man zweckmäßigerweise eine 75 bis 100 gew.-%ige, vorzugsweise 85 bis 98 gew.-%ige Säure (sog. "konzentrierte Schwefelsäure" oder "Schwefelsäure Monohydrat"). Die Veresterung kann in einem Lösungs- oder Verdünnungsmittel vorgenommen werden, wenn es für die Steuerung der Reaktion, z. B. die Wärmeentwicklung erwünscht ist. In der Regel wird der alkoholische Reaktant vorgelegt und das Sulfatierungsmittel unter ständigem Durchmischen allmählich zugefügt. Wenn eine vollständige Veresterung der Alkoholkomponente gewünscht wird, verwendet man das Sulfatierungsmittel und die Alkoholkomponente vorzugsweise im Molverhältnis von 1 : 1 bis 1 : 1.5, besonders bevorzugt von 1 : 1 bis 1 : 1. 2. Die Veresterung wird vorzugsweise bei Temperaturen von 20 bis 90 °C, besonders bevorzugt 45 bis 75 °C, durchgeführt. Gegebenenfalls kann es zweckmäßig sein, die Veresterung in einem niedrig siedenden, mit Wasser nicht mischbaren Lösungs- und Verdünnungsmittel bei dessen Siedepunkt auszuführen, wobei das bei der Veresterung entstehende Wasser azeotrop abdestilliert wird.

### Schritt c)

Als Base für die Neutralisation können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxid, Calciumoxid, Magnesiumhydroxid oder Magnesiumcarbonat sowie Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Trüsopropylamin. Bevorzugt sind Aminoalkohole, z. B. Trialkanolamine, wie Triethanolamin, Alkyldialkanolamine, wie Methyl- oder Ethyldiethanolamin und Dialkylalkanolamine, wie Dimethylethanolamin sowie 2-Amino-2-methyl-1-propanol. Die Neutralisation der Säuregruppen kann auch mit Hilfe von Mischungen mehrerer Basen vorgenommen werden. Besonders bevorzugt ist die Base ausgewählt unter NaOH, KOH, 2-Amino-2-methyl-1-propanol, Triethylamin, Diethylaminopropylamin, Diethanolamin, Triethanolamin, Triisopropanolamin und Mischungen davon. Die Neutralisation kann je nach Anwendungszweck partiell oder vollständig erfolgen. Für eine partielle Neutralisation können z. B. 50 bis weniger als 100 % der Säuregruppen neutralisiert werden. Bevorzugt ist die vollständige Neutralisation.

Die Verbindungen der allgemeinen Formel (I) und der allgemeinen Formel (I.1) eignen sich in vorteilhafter Weise für den Einsatz als anionische Tenside. Es kann sich ganz allgemein beispielsweise um kosmetische Zusammensetzungen, pharmazeutische Zusammensetzungen, Hygieneprodukte, Wasch- und Reinigungsmittel, Anstrichmittel, Zusammensetzungen für die Papierindustrie, Zusammensetzungen für die Textilindustrie, etc. handeln.

Die erfindungsgemäßen Tenside können dabei als alleinige oberflächenaktive Substanz eingesetzt werden. Vorteilhafterweise zeichnen sich die Verbindungen der allgemeinen Formel (I) und der allgemeinen Formel (I.1) durch ihre gute Verträglichkeit mit weiteren Tensiden aus.

### Tensidhaltige Zusammensetzungen

Die erfindungsgemäßen Verbindungen der allgemeinen Formel (I) bzw. der allgemeinen Formel (I.1) eignen sich besonders vorteilhaft zur Formulierung tensidhaltiger Zusammensetzungen. Insbesondere handelt es sich dabei um wässrige tensidhaltige Zusammensetzungen. Die Verbindungen (I) bzw. (I.1) zeichnen sich in derartigen Zusammensetzungen durch ihre gute Wasserlöslichkeit, eine gute Verträglichkeit mit vielen der in der Kosmetik eingesetzten Wirk- und Hilfsstoffen, ein gutes Schaumbildungsvermögen und ein gutes Rheologieverhalten aus.

Die erfindungsgemäßen tensidhaltigen Zusammensetzungen weisen vorzugsweise einen Gesamttensidgehalt von 0,1 bis 75 Gew.-%, besonders bevorzugt von 0,5 bis 60 Gew.-%, insbesondere von 1 bis 50 Gew.-%, bezogen auf das Gesamtgewicht der tensidhaltigen Zusammensetzung, auf.

Geeignete Tenside sind von den Verbindungen (I) verschiedene anionische Tenside, nichtionische Tenside, kationische Tenside, amphotere Tenside und Mischungen davon.

Typische Beispiele für anionische Tenside sind Seifen, Alkylsulfonate, Alkylbenzolsulfonate, Olefinsulfonate, Alkylethersulfonate, Glycerinethersulfonate, Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acylglutamate und Acylaspartate, sowie Acyllactylate, Acyltartrate, Alkyloligoglucosidsulfate, Alkylglucosecarboxylate, Proteinfettsäurekondensate und Alkyl(ether)phosphate.

Geeignete Seifen sind z. B. Alkali-, Erdalkali- und Ammoniumsalze von Fettsäuren, wie Kaliumstearat.

Geeignete Olefinsulfonate werden z. B. durch Anlagerung von SO₃ an Olefine der Formel R³-CH=CH-R⁴ und nachfolgende Hydrolyse und Neutralisation erhalten, wobei R³ und R⁴ unabhängig voneinander für H oder Alkylreste mit 1 bis 20 Kohlenstoffatomen stehen, mit der Maßgabe, dass R³ und R⁴ zusammen mindestens 6 und vorzugsweise 8 bis 20, speziell 10 bis 16, Kohlenstoffatome aufweisen. Hinsichtlich Herstellung und Verwendung sei auf den Übersichtsartikel "J. Am. Oil. Chem. Soc.", 55, 70 (1978) verwiesen. Die Olefinsulfonate können als Alkali-, Erdalkali-, Ammonium-, Alkylammonium-, Alkanolammonium- oder Glucammoniumsalze vorliegen. Vorzugsweise liegen die Olefinsulfonate als Natriumsalze vor. Das hydrolysierte alpha-Olefinsulfonierungsprodukt, d. h. die alpha-Olefinsulfonate, setzen sich aus ca. 60 Gew.-% Alkansulfonaten und ca. 40 Gew.-% Hydroxyalkansulfonaten zusammen; hiervon sind etwa 80 bis 85 Gew.-% Mono- und 15 bis 20 Gew.-% Disulfonate.

Bevorzugte Methylestersulfonate (MES) werden durch Sulfonierung der Fettsäuremethylester von pflanzlichen oder tierischen Fetten oder Ölen gewonnen. Bevorzugt sind Methylestersulfonate aus pflanzlichen Fetten und Ölen, z. B. aus Rapsöl, Sonnenblumenöl, Sojaöl, Palmöl, Kokosfett, etc.

Bevorzugte Alkylsulfate sind Sulfate von Fettalkoholen der allgemeinen Formel R⁶-O-SO₃Y, worin R⁶ für einen linearen oder verzweigten, gesättigten oder ungesättigten Kohlenwasserstoffrest mit 6 bis 22 Kohlenstoffatomen und Y für ein Alkalimetall, das einwertige Ladungsäquivalent eines Erdalkalimetalls, Ammonium-, Mono-, Di-, Tri-oder Tetraalkylammonium, Alkanolammonium oder Glucammonium steht. Geeignete Fettalkoholsulfate werden vorzugsweise durch Sulfatierung von nativen Fettalkoholen oder synthetischen Oxoalkoholen und nachfolgender Neutralisation erhalten werden. Typische Beispiele für Fettalkoholsulfate sind die Sulfatierungsprodukte des Capronalkohols, Caprylalkohols, 2-Ethylhexylalkohols, Caprinalkohols, Laurylalkohols, Isotridecylalkohols, Myristylalkohols, Cetylalkohols, Palmoleylalkohols, Stearylalkohols, Isostearylalkohols, Oleylalkohols, Elaidylalkohols, Petroselinylalkohols, Linolylalkohols, Linolenylalkohols, Behenylalkohols und Elaeostearylalkohols sowie die Salze und Mischungen davon. Bevorzugte Salze der Fettalkoholsulfate sind die Natrium- und Kaliumsalze, insbesondere die Natriumsalze. Bevorzugte Mischungen der Fettalkoholsulfate basieren auf technischen Alkoholmischungen, die z. B. bei der Hochdruckhydrierung von technischen Methylestern auf Basis von Fetten und Ölen oder bei der Hydrierung von Aldehyden aus der Oxosynthese oder bei der Dimerisierung von ungesättigten Fettalkoholen anfallen. Vorzugsweise werden zur Herstellung von Alkylsulfaten Fettalkohole und Fettalkoholgemische mit 12 bis 18 Kohlenstoffatomen und insbesondere 12 bis 14 Kohlenstoffatomen eingesetzt. Typische Beispiele hierfür sind technische Alkoholsulfate auf Basis von pflanzlichen Rohstoffen.

Bevorzugte Sarkosinate sind Natriumlauroylsarkosinat oder Natriumstearoylsarkosinat.

Bevorzugte Proteinfettsäurekondensate sind pflanzliche Produkte auf Weizenbasis.

Bevorzugte Alkylphosphate sind Mono- und Diphosphorsäurealkylester.

Geeignete Acylglutamate sind Verbindungen der Formel (I) 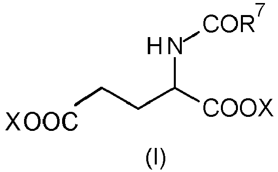 worin COR⁷ für einen linearen oder verzweigten Acylrest mit 6 bis 22 Kohlenstoffatomen und 0, 1, 2 oder 3 Doppelbindungen und X für Wasserstoff, ein Alkalimetall, das einwertige Ladungsäquivalent eines Erdalkalimetalls, Ammonium, Alkylammonium, Alkanolammonium oder Glucammonium steht. Die Herstellung von Acylglutamaten erfolgt beispielsweise durch Schotten-Baumann-Acylierung von Glutaminsäure mit Fettsäuren, Fettsäureestern oder Fettsäurehalogeniden. Acylglutamate sind kommerziell beispielsweise von der BASF SE, Clariant AG, Frankfurt/DE, oder der Ajinomoto Co. Inc., Tokio/JP, erhältlich. Eine Übersicht zu Herstellung und Eigenschaften der Acylglutamate findet sich von M. Takehara et al. in J. Am. Oil Chem. Soc. 49 (1972) 143. Typische als Komponente b) geeignete Acylglutamate leiten sich bevorzugt von Fettsäuren mit 6 bis 22 und besonders bevorzugt 12 bis 18 Kohlenstoffatomen ab. Es werden insbesondere die Mono- oder Dialkalimetallsalze des Acylglutamats eingesetzt. Dazu zählen z. B. (Handelsnamen der Fa. Ajinomoto, USA in Klammern): Natriumcocoylglutamat (Amisoft CS-11), Dinatriumcocoylglutamat (Amisoft ECS-22SB), Triethanolammoniumcocoylglutamat (Amisoft CT-12), Triethanolammoniumlauroylglutamat (Amisoft LT-12), Natriummyristoylglutamat (Amisoft MS-11), Natriumstearoylglutamat (Amisoft HS-11 P) und Mischungen davon.

Zu den nichtionischen Tensiden gehören beispielsweise:
- Fettalkoholpolyoxyalkylenester, beispielsweise Laurylalkoholpolyoxyethylenacetat,
- Alkylpolyoxyalkylenether, die sich von niedermolekularen C₁-C₆-Alkoholen oder von C₇-C₃₀-Fettalkoholen ableiten. Dabei kann die Etherkomponente aus Ethylenoxideinheiten, Propylenoxideinheiten, 1,2-Butylenoxideinheiten, 1,4-Butylenoxideinheiten und statistischen Copolymeren und Blockcopolymeren davon abgeleitet sein. Dazu zählen speziell Fettalkoholalkoxylate und Oxoalkoholalkoxylate, insbesondere vom Typ
   RO-(R⁸O)r(R⁹O)sR¹⁰ mit R⁸ und R⁹ unabhängig voneinander = C₂H₄, C₃H₆, C₄H₈ und R¹⁰ = H, oder C₁-C₁₂-Alkyl, R = C₃-C₃₀-Alkyl oder C₆-C₃₀-Alkenyl, r und s unabhängig voneinander 0 bis 50, wobei nicht beide für 0 stehen können, wie iso-Tridecylalkohol- und Oleylalkoholpolyoxyethylenether,
- Alkylarylalkohol-polyoxyethylenether, z. B. Octylphenol-polyoxyethylenether,
- alkoxylierte tierische und/oder pflanzliche Fette und/oder Öle, beispielsweise Maisölethoxylate, Rizinusölethoxylate, Talgfettethoxylate,
- Glycerinester, wie beispielsweise Glycerinmonostearat,
- Alkylphenolalkoxylate, wie beispielsweise ethoxyliertes iso-Octyl-, Octyl- oder Nonylphenol, Tributylphenolpolyoxyethylenether,
- Fettaminalkoxylate, Fettsäureamid- und Fettsäurediethanolamidalkoxylate, insbesondere deren Ethoxylate,
- Zuckertenside, Sorbitester, wie beispielsweise Sorbitanfettsäureester (Sorbitanmonooleat, Sorbitantristearat), Polyoxyethylensorbitanfettsäureester, Alkylpolyglycoside, N-Alkylgluconamide,
- Alkylmethylsulfoxide,
- Alkyldimethylphosphinoxide, wie beispielsweise Tetradecyldimethylphosphinoxid.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate. Beispielsweise können Cocodimethylsulfopropylbetain, Laurylbetain, Cocamidopropylbetain, Natriumcocamphopropionat oder Tetradecyldimethylaminoxid eingesetzt werden.

Zu den kationischen Tensiden gehören beispielsweise quaternierte Ammoniumverbindungen, insbesondere Alkyltrimethylammonium- und Dialkyldimethylammoniumhalogenide und -alkylsulfate sowie Pyridin- und Imidazolin-Derivate, insbesondere Alkylpyridinium-Halogenide. Beispielsweise können Behenyl oder Cetyltrimethylammoni-umchlorid eingesetzt werden. Geeignet sind weiterhin sogenannte Esterquats, die auf quartären Triethanol-Methyl-Ammonium- oder quartären Diethanol-Dimethyl-Ammonium-Verbindungen mit langen Kohlenwasserstoffketten in Form von Fettsäureestern basieren. Dazu zählt beispielsweise Bis(acyloxyethyl)hydroxyethylammonium Methosulfat. Geeignet ist weiterhin Dehyquart L 80 (INCI: Dicocoylethyl Hydroxyethylmonium Methosulfate (and) Propylene Glycol).

### Kosmetische und pharmazeutische Mittel

Die Verbindungen der allgemeinen Formel (I) und der allgemeinen Formel (I.1) eignen sich vorzugsweise zur Formulierung von kosmetischen und pharmazeutischen Produkten, speziell von wässrigen kosmetischen und pharmazeutischen Produkten.

Ein weiterer Gegenstand der Erfindung ist eine kosmetische oder pharmazeutische Zusammensetzung, enthaltend
a) wenigstens eine Verbindung der allgemeinen Formel (I), wie zuvor definiert,
b) wenigstens einen kosmetischen oder pharmazeutischen Wirkstoff, und
c) gegebenenfalls wenigstens einen von den Komponenten a) und b) verschiedenen kosmetischen oder pharmazeutischen Hilfsstoff.

Bevorzugt wird als Komponente a) wenigstens eine Verbindung der allgemeinen Formel (I.1) eingesetzt.

Bevorzugt umfasst die Komponente c) wenigstens einen kosmetischen oder pharmazeutischen Träger.

Vorzugsweise ist die Träger-Komponente c) ausgewählt unter
i) Wasser,
ii) wassermischbaren organischen Lösungsmitteln, vorzugsweise C₂-C₄-Alkanolen, insbesondere Ethanol,
iii) Ölen, Fetten, Wachsen,
iv) von iii) verschiedenen Estern von C₆-C₃₀-Monocarbonsäuren mit ein-, zwei- oder dreiwertigen Alkoholen,
v) gesättigten acyclischen und cyclischen Kohlenwasserstoffen,
vi) Fettsäuren,
vii) Fettalkoholen,
viii) Treibgasen,

### und Mischungen davon.

Speziell geeignete kosmetisch verträgliche Öl- bzw. Fettkomponenten c) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Bei den erfindungsgemäßen kosmetischen Mitteln kann es sich um hautkosmetische, haarkosmetische, dermatologische, hygienische oder pharmazeutische Mittel handeln. Aufgrund ihrer grenzflächenaktiven Eigenschaften eignen sich die zuvor beschriebenen Verbindungen der Formel (I) und der Formel (I.1) insbesondere in Zusammensetzungen zur Reinigung der Haut und/oder der Haare.

Vorzugsweise liegen die erfindungsgemäßen Mittel in Form einer wässrigen Lösung, einer festen Formulierung (z. B. eines Seifenstücks oder Waschsticks), eines Schaums, einer Emulsion, Suspension, Lotion, eines Gels, einer Paste oder eines Sprays, vor. Gewünschtenfalls können auch Liposomen oder Mikrosphären eingesetzt werden.

Die erfindungsgemäßen kosmetischen Mittel können zusätzlich kosmetische und/oder dermatologische Wirk- und Effektstoffe sowie Hilfsstoffe enthalten. Vorzugsweise enthalten die erfindungsgemäßen kosmetischen Mittel wenigstens eine Verbindung der Formel (I) oder der Formel (I.1), wie vorstehend definiert, wenigstens einen wie vorstehend definierten Träger C) und wenigstens einen davon verschiedenen Bestandteil, der vorzugsweise ausgewählt ist unter kosmetischen Wirkstoffen, Emulgatoren, Tensiden, Konservierungsmitteln, Parfümölen, zusätzlichen Verdickern, Haarpolymeren, Haar- und Hautconditionern, Pfropfpolymeren, wasserlöslichen oder dispergierbaren silikonhaltigen Polymeren, Lichtschutzmitteln, Bleichmitteln, Gelbildnern, Pflegemitteln, Tönungsmitteln, Bräunungsmitteln, Farbstoffen, Pigmenten, Konsistenzgebern, Feuchthaltemitteln, Rückfettern, Collagen, Eiweißhydrolysaten, Lipiden, Antioxidantien, Entschäumern, Antistatika, Emollienzien und Weichmachern.

Zusätzlich zu den Verbindungen der Formel (I) und der Formel (I.1) können die kosmetischen Mittel wenigstens ein Verdickungsmittel enthalten. Dazu zählen z. B. Polysaccharide und organische Schichtmineralien wie Xanthan Gum® (Kelzan® der Fa. Kelco), Rhodopol® 23 (Rhone Poulenc) oder Veegum® (Firma R. T. Vanderbilt) oder Attaclay® (Firma Engelhardt). Geeignete Verdickungsmittel sind auch organische natürliche Verdickungsmittel (Agar-Agar, Carrageen, Tragant, Gummi arabicum, Alginate, Pektine, Polyosen, Guar-Mehl, Johannisbrotbaumkernmehl, Stärke, Dextrine, Gelatine, Casein) und anorganische Verdickungsmittel (Polykieselsäuren, Tonmineralien wie Montmorillonite, Zeolithe, Kieselsäuren). Weitere Verdickungsmittel sind Polysaccharidgummen, beispielsweise Gummi arabicum, Agar, Alginate, Carrageene und ihre Salze, Guar, Guaran, Traganth, Gellan, Ramsan, Dextran oder Xanthan und ihre Derivate, z. B. propoxyliertes Guar, sowie ihre Mischungen. Andere Polysaccharidverdicker sind beispielsweise Stärken verschiedensten Ursprungs und Stärkederivate, z. B. Hydroxyethylstärke, Stärkephosphatester oder Stärkeacetate oder Carboxymethylcellulose bzw. ihr Natriumsalz, Methyl-, Ethyl-, Hydroxyethyl-, Hydroxypropyl-, Hydroxypropylmethyl- oder Hydroxyethyl-methyl-cellulose oder Celluloseacetat. Als Verdickungsmittel können weiterhin Schichtsilikate eingesetzt werden. Hierzu zählen beispielsweise die unter dem Handelsnamen Laponite ® erhältlichen Magnesium-oder Natrium-Magnesium-Schichtsilikate der Firma Solvay Alkali sowie die Magnesiumsilikate der Firma Süd-Chemie.

Geeignete kosmetische und/oder dermatologische Wirkstoffe sind z. B. Haut- und Haarpigmentierungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B-und/oder UV-A-Bereich absorbieren. Geeignete UV-Filter sind die zuvor genannten.

Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend wirkende Stoffe, welche die Durchblutung der Haut anregen, sind z. B. ätherische Öle, wie Latschenkiefer, Lavendel, Rosmarin, Wacholderbeere, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Die erfindungsgemäßen kosmetischen Mittel können als kosmetischen Wirkstoff (wie auch gegebenenfalls als Hilfsstoff) wenigstens ein kosmetisches oder pharmazeutisches Polymer enthalten. Dazu zählen ganz allgemein anionische, kationische, amphotere und neutrale Polymere.

Beispiele für anionische Polymere sind Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luvimer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. C₄-C₃₀-Alkylester der (Meth)acrylsäure, C₄-C₃₀-Alkylvinylester, C₄-C₃₀-Alkylvinylether und Hyaluronsäure. Ein Beispiel für ein anionisches Polymer ist weiterhin das unter der Bezeichnung Luviset® Shape (INCI Name: Polyacrylate-22) erhältliche Methylmethacrylat/Methacrylsäure/Acrylsäure/Urethanacrylat-Copolymer. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und - R1 vertrieben werden und die Carboset®-Marken der Fa. BF Goodrich.

Geeignete kationische Polymere sind z. B. kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviset Clear ®, Luviquat Supreme ®, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Ganz besonders geeignete Polymere sind neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF SE), oder Polyvinylpyrrolidon und deren Copolymere, insbesondere mit Vinylestern, wie Vinylacetat, z. B. Luviskol® VA 37, VA 55, VA 64, VA 73 (BASF SE); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methyl-methacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

In einer speziellen Ausführung enthalten die erfindungsgemäßen Zusammensetzungen wenigstens ein Polymer, das als Verdicker wirkt.

Geeignete polymere Verdicker sind beispielsweise gegebenenfalls modifizierte polymere Naturstoffe (Carboxymethylcellulose und andere Celluloseether, Hydroxyethyl- und -propylcellulose und dergleichen) sowie synthetische polymere Verdicker (Polyacryl-und Polymethacryl-Verbindungen, Vinylpolymere, Polycarbonsäuren, Polyether, Polyimine, Polyamide). Dazu zählen die zum Teil bereits zuvor genannten Polyacryl- und Polymethacryl-Verbindungen, beispielsweise die hochmolekularen mit einem Polyalkenylpolyether, insbesondere einem Allylether von Saccharose, Pentaerythrit oder Propylen, vernetzten Homopolymere der Acrylsäure (INCI-Bezeichnung: Carbomer). Solche Polyacrylsäuren sind u. a. von der Fa. BF Goodrich unter dem Handelsnamen Carbopol ® erhältlich, z. B. Carbopol 940 (Molekulargewicht ca. 4000000), Carbopol 941 (Molekulargewicht ca. 1250000) oder Carbopol 934 (Molekulargewicht ca. 3000000). Weiterhin fallen darunter Acrylsäure-Copolymere, die beispielsweise von der Fa. Rohm & Haas unter den Handelsnamen Aculyn ® und Acusol ® erhältlich sind, z. B. die anionischen, nicht-assoziativen Polymere Aculyn 22, Aculyne 28, Aculyn 33 (vernetzt), Acusol 810, Acusol 823 und Acusol 830 (CAS 25852-37-3). Speziell geeignet sind auch assoziative Verdicker, z.B. auf Basis modifizierter Polyurethane (HEUR) oder hydrophob modifizierter Acryl- oder Methacrylsäure-Copolymere (HASE-Verdicker, High Alkali Swellable Emulsion).

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Hautreinigungsmittel enthalten vorzugsweise wenigstens eine Verbindungen der Formel (I) und der Formel (I.1) in einem Anteil von etwa 0,001 bis 70 Gew.-%, vorzugs-weise 0,01 bis 50 Gew.-%, ganz besonders bevorzugt 0,1 bis 30 Gew.-%, bezogen auf das Gesamtgewicht des Mittels.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens eine Verbindung der allgemeinen Formel (I) oder (I.1) als Basistensid und gegebenenfalls wenigstens ein amphoteres und/oder nichtionisches Tensid als Cotensid. Weitere geeignete Wirkstoffe und/oder Hilfsstoffe sind im Allgemeinen ausgewählt unter Lipiden, Parfümölen, Farbstoffen, organischen Säuren, Konservierungsstoffen, Antioxidantien, Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können zusätzlich alle in Körperreinigungsmitteln üblicherweise eingesetzten anionischen, neutralen, amphoteren oder kationischen Tenside verwendet werden.

Geeignete Tenside sind die zuvor genannten.

Weiterhin können die Duschgel-/Shampoo-Formulierungen zusätzliche Verdicker, wie z. B. Kochsalz, PEG-55, Propylenglykol-Oleat, PEG-120-Methylglucosedioleat und andere enthalten. Geeignete kommerziell erhältliche weitere Verdicker sind z. B. Arlypon TT (INCI: PEG/PPG-120/10 Trimethylolpropane Trioleate (and) Laureth-2) und Arlypon F (INCI: Laureth-2). Des Weiteren können die Duschgel-/ShampooFormulierungen Konservierungsmittel, weitere Wirk- und Hilfsstoffe und Wasser enthalten.

Die erfindungsgemäßen Verbindungen der Formel (I) und der Formel (I.1) eignen sich auch vorteilhaft als Tenside für Shampooformulierungen, die zusätzlich weitere übliche Tenside enthalten können.

In den Shampooformulierungen können zur Erzielung bestimmter Effekte übliche Konditioniermittel eingesetzt werden. Hierzu zählen beispielsweise die zuvor genannten kationischen Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Außerdem können die Verbindungen der Formel (I) und der Formel (I.1) verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Die hautkosmetischen Zubereitungen können neben den Verbindungen der Formel (I) und der Formel (I.1) und geeigneten Trägern noch weitere in der Hautkosmetik übliche Wirkstoffe und Hilfsstoffe, wie zuvor beschrieben, enthalten.

Bevorzugte Öl- und Fettkomponenten der hautkosmetischen und der dermatologischen Mittel sind mineralische und synthetische Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von C₆-C₃₀-Fettsäuren, Wachsester, wie z. B. Jojobaöl, Fettalkohole, Vaseline, hydriertes Lanolin und acetyliertes Lanolin sowie Mischungen davon.

Zur Einstellung bestimmter Eigenschaften wie z. B. Verbesserung des Anfassgefühls, des Spreitverhaltens, der Wasserresistenz und/oder der Bindung von Wirk- und Hilfsstoffen, wie Pigmenten, können die hautkosmetischen und dermatologischen Zubereitungen zusätzlich auch konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung von kosmetischen oder dermatologischen Zubereitungen, die wenigstens eine Verbindung der Formel (I) oder der Formel (I.1) enthalten, erfolgt nach üblichen, dem Fachmann bekannten Verfahren.

Bevorzugt liegen die kosmetischen und dermatologischen Mittel in Form von Emulsionen, insbesondere als Wasser-in-ÖI-(W/O)- oder Öl-in-Wasser-(O/W)-Emulsionen, vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einer Verbindung der Formel (I) oder der Formel (I.1) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-ÖI; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl-oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Es können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens eine Verbindung der allgemeinen Formel (I) oder (I.1) in einer Menge im Bereich von etwa 0,1 bis 30 Gew.-%, bevorzugt 0,5 bis 20 Gew.-%, bezogen auf das Gesamtgewicht des Mittels.

Die erfindungsgemäßen Verbindungen der Formel (I) und der Formel (I.1) eignen sich auch für Styling-Gele. Als Gelbildner können alle in der Kosmetik üblichen Gelbildner eingesetzt werden. Hierzu wird auf die zuvor genannten konventionellen Verdicker Bezug genommen.

Die erfindungsgemäßen Verbindungen der Formel (I) und der Formel (I.1) eignen sich ebenso zur Herstellung von pharmazeutischen Zusammensetzungen.

Geeignete pharmazeutische Hilfsstoffe sind die in einschlägigen Arzneibüchern (z. B. DAB, Ph. Eur., BP, NF) gelisteten Hilfsstoffe sowie andere Hilfsstoffe, deren Eigenschaften einer physiologischen Anwendung nicht entgegenstehen. Derartige Stoffe sind beispielsweise auch in Fiedler, H. P. Lexikon der Hilfsstoffe für Pharmazie, Kosmetik und angrenzende Gebiete, 4. Aufl., Aulendorf: ECV-Editio-Kantor-Verlag, 1996, beschrieben.

### Wasch- und Reinigungsmittel

Die erfindungsgemäßen Verbindungen der Formel (I) und der Formel (I.1) eignen sich auch zur Herstellung von Wasch- oder Reinigungsmittel, z. B. zum Reinigen von textilen Flächengeweben und/oder harten Oberflächen. Derartige Reinigungsmittel können in Form eines Hand- oder Maschinengeschirrspülmittels, Allzweckreiniger für nichttextile Oberflächen, z. B. aus Metall, lackiertem Holz oder Kunststoff, oder Reinigungsmittel für keramische Erzeugnisse, wie Porzellan, Fliesen, Kacheln vorliegen. Bevorzugt liegen die erfindungsgemäßen Wasch- oder Reinigungsmittel in Form eines flüssigen Textilwaschmittels vor. Diese können gewünschtenfalls auch pastös formuliert werden.

Beispiele für weitere Formulierungen, in denen wenigstens eine Verbindung der allgemeinen Formel (I) oder der Formel (I.1) wie zuvor definiert, vorteilhaft eingesetzt werden kann, sind z. B.

Allzweckreiniger, Sprühreiniger, Handgeschirrspülmittel zur Reinigung im privaten, industriellen und institutionellen Bereich;
Feuchthaltemittel;
Druckwalzen- und Druckplattenreinigungsmittel in der Druckindustrie;
Lacke und Farbformulierungen;
Beschichtungsmittel, z. B. für Papier;
Klebstoffe;
Formulierungen für Sprühanwendungen, zum Beispiel in Ink jet-Tinten;
Lederbehandlungsmittel;
Mittel zur Metallbehandlung, wie Korrosionsschutzformulierungen;
Schneid-, Schleif-oder Bohrhilfsmittel und Schmiermittel;
Formulierungen in der Textilindustrie, wie Egalisiermittel oder Formulierungen zur Garnreinigung;
Flotationshilfsmittel und Schäumhilfsmittel.

Solche Formulierungen enthalten üblicherweise weitere Inhaltsstoffe wie Tenside, Gerüst-, Duft- und Farbstoffe, Komplexbildner, Polymere und andere Inhaltsstoffe. Allgemein können die erfindungsgemäßen Verbindungen der allgemeinen Formel (I) in allen Bereichen eingesetzt werden, in denen die eine verdickende grenzflächenaktive Wirkung notwendig ist. Weiterhin sind die Verbindungen der allgemeinen Formel (I) geeignet, die Löslichkeit anderer Komponenten, z. B. anderer oberflächenaktiver Komponenten, wie von anionischen Tensiden, zu verbessern. Sie tragen somit auch positiv zur Bildung klarer tensidhaltiger Lösungen bei.

Die Erfindung wird anhand der folgenden nicht einschränkenden Beispiele näher erläutert.

### Beispiele

### Beispiel 1:

In einem kontinuierlich arbeitenden Fallfilmreaktor mit Mantelkühlung und SO₃-Begasung wurden 600 g eines technischen Lauryloligolactats (68 % Monoester) bei 40 °C mit Schwefeltrioxid umgesetzt. Das Molverhältnis betrug 1,2 mol SO₃ pro Mol Lauryloligolactat. Das Schwefeltrioxid wurde durch Erhitzen aus einer entsprechenden Menge 65 gew.-%igen Oleums ausgetrieben, mit Stickstoff auf eine Konzentration von 5 Vol.-% verdünnt und über eine Düse mit dem Lauryllactatfilm in Kontakt gebracht. Anschließend wurde es zusammen mit 50 gew.-%iger Natriumhydroxidlösung in eine 1 gew.-%ige Lösung von Kaliumdihydrogenphosphat eingerührt und bei einem pH von 5,5 bis 7,5 neutralisiert.

### Analytik:

| | | |
|---|---|---|
| Aniontensidgehalt | 14,5% | (MG=374,4 g/mol) |
| Unsulfiertes: | 2,47 % | |
| Wasser nach Karl Fischer: | 83,9 % | |
| Na₂SO₄: | 1,7 % | |

### Vergleichsbeispiel:

In einem kontinuierlich arbeitenden Fallfilmreaktor mit Mantelkühlung und SO₃-Begasung wurden 600 g eines Lauryllactats (87 % Monoester) bei 40 °C mit Schwefeltrioxid umgesetzt. Das Molverhältnis betrug 1,2 mol SO₃ pro Mol Lauryllactat. Das Schwefeltrioxid wurde durch Erhitzen aus einer entsprechenden Menge 65 gew.-%igen Oleums ausgetrieben, mit Stickstoff auf eine Konzentration von 5 Vol.-% verdünnt und über eine Düse mit dem Lauryllactatfilm in Kontakt gebracht. Anschließend wurde es zusammen mit 50 gew.-%iger Natriumhydroxidlösung in eine 1 gew.-%ige Lösung von Kaliumdihydrogenphosphat eingerührt und bei einem pH von 5,5 bis 7,5 neutralisiert.

### Analytik

| | | |
|---|---|---|
| Aniontensidgehalt: | 15,4 % | (MG=368,5 g/mol) |
| Unsulfiertes: | 1,3 % | |
| Wasser nach Karl Fischer: | 82,8 % | |
| Na₂SO₄: | 1,72 % | |

### Anwendungstest:

Die Substanz aus Beispiel 1 und die Vergleichssubstanz wurden auf eine Konzentration von 12 % eingestellt und steigende Mengen an NaCl zugegeben. Bestimmt wurde die Viskosität der Lösungen mit einem Viskosimeter: Brookfield DII + pro bei einer Messtemperatur von 22 °C.

| | Beispiel 1 | Vergleichsbeispiel |
|---|---|---|
| NaCl Menge | Viskosität [mPas] | |
| 0,5 | 280 | 120 |
| 1 | 1500 | 760 |
| 1,5 | 4800 | 3700 |
| 2 | 4700 | 4500 |

Das erfindungsgemäße Beispiel zeigt einen höheren Viskositätsanstieg bei niedrigeren NaCl Gehalten.

Weiterhin wurde das Schaumverhalten der beiden Substanzen mit einem Sita Rotorschaum-Messgerät (1,0 g/l, 30 °C, 1300 rpm; pH = 5,5) geprüft.

| | Beispiel 1 | Vergleichsbeispiel |
|---|---|---|
| Rührzeit [s] | Schaumvolumen [ml] | |
| 0 | 0 | 0 |
| 10 | 231 | 237 |
| 20 | 392 | 400 |
| 30 | 532 | 539 |
| 40 | 765 | 765 |
| 50 | 829 | 828 |
| 60 | 851 | 849 |
| 70 | 860 | 857 |
| 80 | 866 | 861 |

Das Schaumverhalten beider Substanzen ist im Rahmen der Messgenauigkeit identisch.

## Patentansprüche

1. Verbindungen der allgemeinen Formel (I) 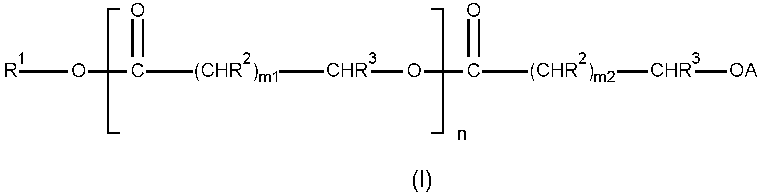 worin
R¹ für Wasserstoff oder einen linearen oder verzweigten aliphatischen Kohlenwasserstoffrest mit 1 bis 30 Kohlenstoffatomen und 0, 1, 2 oder 3 Doppelbindungen steht,
die Reste R² unabhängig voneinander ausgewählt sind unter Wasserstoff, Methyl, Ethyl, -OA, -COOR⁴, -CH₂-OA und -CH₂-COOR⁴, wobei die Reste R⁴ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
die Reste R³ unabhängig voneinander ausgewählt sind unter Wasserstoff, Methyl, Ethyl, -OA, -COOR⁵, -CH₂-OA und -CH₂-COOR⁵, wobei die Reste R⁵ für Wasserstoff oder einen linearen oder verzweigten aliphatischen Kohlenwasserstoffrest mit 1 bis 30 Kohlenstoffatomen und 0, 1, 2 oder 3 Doppelbindungen stehen,
A für H oder -SO₃B steht, worin B für Wasserstoff oder ein Kationenäquivalent steht,
n im Mittel für einen Wert von wenigstens 0,1 steht,
m1 und m2 unabhängig voneinander für 0 oder 1 stehen,
mit der Maßgabe, dass wenigstens einer der Reste A für -SO₃B steht, und
mit der Maßgabe, dass wenigstens einer der Reste R¹, R⁴ oder R⁵ für einen linearen oder verzweigten aliphatischen Kohlenwasserstoffrest mit 1 bis 30 Kohlenstoffatomen und 0, 1, 2 oder 3 Doppelbindungen steht.

2. Verbindungen (I) nach Anspruch 1, wobei m1 und m2 die gleiche Bedeutung haben.

3. Verbindungen (I) nach einem der vorhergehenden Ansprüche, die abgeleitet sind von Milchsäure, Glycolsäure, Äpfelsäure, Weinsäure oder Mischungen davon.

4. Verbindungen (I) nach einem der vorhergehenden Ansprüche, die ausgewählt sind unter Verbindungen der Formel (I.1) 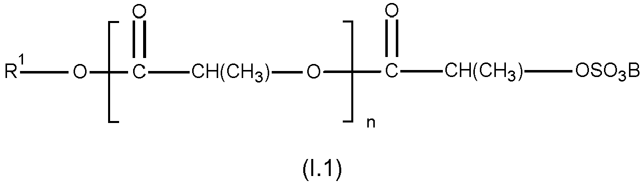 worin
R¹ für Wasserstoff oder einen linearen oder verzweigten Kohlenwasserstoffrest mit 6 bis 30 Kohlenstoffatomen und 0, 1, 2 oder 3 Doppelbindungen steht,
B für Wasserstoff oder ein Kationenäquivalent steht, und
n im Mittel für einen Wert von wenigstens 0,1 steht.

5. Verbindungen (I) nach einem der vorhergehenden Ansprüche, worin n für einen Wert von 0,1 bis 100, vorzugsweise von 0,15 bis 50, insbesondere von 0,2 bis 20, steht.

6. Verbindungen (I) nach einem der vorhergehenden Ansprüche, wobei wenigstens einer der Reste R¹, R⁴ und R⁵ für Methyl, Ethyl, n-Propyl, Isopropyl, n-Butyl, Isobutyl, sek.-Butyl, tert.-Butyl, n-Pentyl, Isoamyl, n-Hexyl, 2-Ethylhexyl, n-Heptyl, n-Octyl, n-Nonyl, n-Decyl, 2-Propylheptyl, n-Undecyl, n-Dodecyl, n-Tridecyl, n-Tetradecyl, n-Pentadecyl, n-Hexadecyl, n-Heptadecyl, n-Octadecyl, n-Nonadecyl, Arachinyl, Behenyl, Lignocerinyl, Melissinyl, Isotridecyl, Isostearyl, Oleyl, Linoleyl, Linolenyl oder eine Kombination aus wenigstens zwei dieser Reste steht.

7. Verbindungen (I) nach einem der vorhergehenden Ansprüche, wobei wenigstens einer der Reste R¹, R⁴ und R⁵ von linearen gesättigten Alkoholen mit 8 bis 18 Kohlenstoffatomen ableitet.

8. Verbindungen (I) nach einem der vorhergehenden Ansprüche, wobei sich wenigstens einer der Reste R¹, R⁴ und R⁵ von einem Gemisch linearer gesättigter C₁₂-/C₁₄-Alkohole ableitet.

9. Verfahren zur Herstellung von Verbindungen der allgemeinen Formel (I), bei dem man
a) wenigstens eine Hydroxycarbonsäure der allgemeinen Formel (I.A) 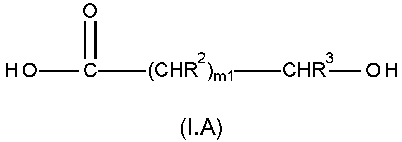 worin R², R³ und m1 wie in einem der Ansprüche 1 bis 8 definiert sind, in einer Veresterungsreaktion umsetzt, wobei die Veresterung in Gegenwart wenigstens eines Alkohols R¹-OH erfolgt, wobei R¹ wie in einem der Ansprüche 1 bis 8 definiert ist oder das Produkt der Veresterung der Hydroxycarbonsäure(n) (I.A) anschließend mit wenigstens einem Alkohol R¹-OH umgesetzt wird,
b) das Reaktionsprodukt aus Schritt a) mit einem Sulfatierungsmittel umsetzt, und
c) gegebenenfalls das Reaktionsprodukt aus Schritt b) mit einer Base zumindest teilweise neutralisiert.

10. Verfahren nach Anspruch 9, wobei das in Schritt b) eingesetzte Sulfatierungsmittel SO₃ umfasst oder aus SO₃ besteht.

11. Kosmetische oder pharmazeutische Zusammensetzung, enthaltend
a) wenigstens eine Verbindung der allgemeinen Formel (I), wie in einem der Ansprüche 1 bis 8 definiert,
b) wenigstens einen kosmetischen oder pharmazeutischen Wirkstoff, und
c) gegebenenfalls wenigstens einen von den Komponenten a) und b) verschiedenen kosmetischen oder pharmazeutischen Hilfsstoff.

12. Kosmetische Zusammensetzung nach Anspruch 11 in Form von Haarshampoos, Duschgelen, Seifen, Syndets, Waschpasten, Waschlotionen, Scrub-Präparate, Schaumbädern, Ölbädern, Duschbädern, Rasierschäumen, Rasierlotionen und Rasiercremes.

13. Wasch- oder Reinigungsmittel, enthaltend wenigstens eine Verbindung der allgemeinen Formel (I), wie in einem der Ansprüche 1 bis 8 definiert.

14. Verwendung wenigstens einer Verbindung der allgemeinen Formel (I), wie in einem der Ansprüche 1 bis 8 definiert, als oberflächenaktive Substanzen.

15. Verwendung wenigstens einer Verbindung der allgemeinen Formel (I), wie in einem der Ansprüche 1 bis 8 definiert, als anionisches Tensid für kosmetische Mittel, als anionisches Tensid für pharmazeutische Mittel, als anionisches Tensid für Wasch- und Reinigungsmittel.

## Claims

1. A compound of the general formula (I) 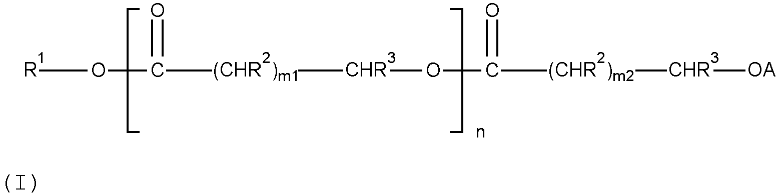 in which
R¹ is hydrogen or a linear or branched aliphatic hydrocarbon radical having 1 to 30 carbon atoms and 0, 1, 2 or 3 double bonds,
the radicals R², independently of one another, are selected from hydrogen, methyl, ethyl, -OA, - COOR⁴, -CH₂-OA and -CH₂-COOR⁴, where the radicals R⁴ are hydrogen or a linear or branched aliphatic hydrocarbon radical having 1 to 30 carbon atoms and 0, 1, 2 or 3 double bonds,
the radicals R³, independently of one another, are selected from hydrogen, methyl, ethyl, -OA, - COOR⁵, -CH₂-OA and -CH₂-COOR⁵, where the radicals R⁵ are hydrogen or a linear or branched aliphatic hydrocarbon radical having 1 to 30 carbon atoms and 0, 1, 2 or 3 double bonds,
A is H or -SO₃B, in which B is hydrogen or a cation equivalent,
n is on average a value of at least 0.1,
m1 and m2, independently of one another, are 0 or 1,
with the proviso that at least one of the radicals A is -SO₃B, and
with the proviso that at least one of the radicals R¹, R⁴ or R⁵ is a linear or branched aliphatic hydrocarbon radical having 1 to 30 carbon atoms and 0, 1, 2 or 3 double bonds.

2. The compound (I) according to claim 1, where m1 and m2 have the same meaning.

3. The compound (I) according to one of the preceding claims, which is derived from lactic acid, glycolic acid, malic acid, tartaric acid or mixtures thereof.

4. The compound (I) according to any one of the preceding claims, which is selected from compounds of the formula (1.1) 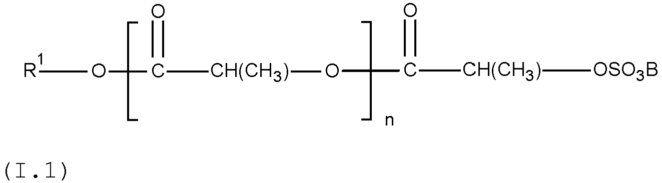 in which
R¹ is hydrogen or a linear or branched hydrocarbon radical having 6 to 30 carbon atoms and 0, 1, 2 or 3 double bonds,
B is hydrogen or a cation equivalent, and
n is on average a value of at least 0.1.

5. The compound (I) according to any one of the preceding claims, in which n is a value from 0.1 to 100, preferably from 0.15 to 50, in particular from 0.2 to 20.

6. The compound (I) according to any one of the preceding claims, where at least one of the radicals R¹, R⁴ and R⁵ is methyl, ethyl, n-propyl, isopropyl, n-butyl, isobutyl, sec-butyl, tert-butyl, n-pentyl, isoamyl, n-hexyl, 2-ethylhexyl, n-heptyl, n-octyl, n-nonyl, n-decyl, 2-propylheptyl, n-undecyl, n-dodecyl, n-tridecyl, n-tetradecyl, n-pentadecyl, n-hexadecyl, n-heptadecyl, n-octadecyl, n-nonadecyl, arachinyl, behenyl, lignocerinyl, melissinyl, isotridecyl, isostearyl, oleyl, linoleyl, linolenyl or a combination of at least two of these radicals.

7. The compound (I) according to any one of the preceding claims, where at least one of the radicals R¹, R⁴ and R⁵ derives from linear saturated alcohols having 8 to 18 carbon atoms.

8. The compound (I) according to any one of the preceding claims, where at least one of the radicals R¹, R⁴ and R⁵ is derived from a mixture of linear saturated C₁₂-/C₁₄-alkohols.

9. A process for preparing compounds of the general formula (I), in which
a) at least one hydroxycarboxylic acid of the general formula (I.A) 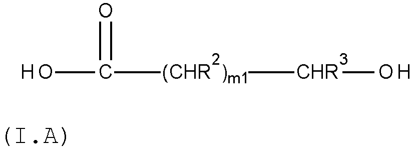 in which R², R³ and m1 are as defined in any one of claims 1 to 8, is reacted in an esterification reaction, where the esterification takes place in the presence of at least one alcohol R¹-OH, where R¹ is as defined in any one of claims 1 to 8, or the product of the esterification of the hydroxycarboxylic acid(s) (I.A) is then reacted with at least one alcohol R¹-OH,
b) the reaction product from step a) is reacted with a sulfating agent, and
c) optionally the reaction product from step b) is at least partially neutralized with a base.

10. The process according to claim 9, where the sulfating agent used in step b) comprises SO₃ or consists of SO₃.

11. A cosmetic or pharmaceutical composition comprising
a) at least one compound of the general formula (I), as defined in any one of claims 1 to 8,
b) at least one cosmetic or pharmaceutical active ingredient, and
c) optionally at least one cosmetic or pharmaceutical auxiliary which is different from components a) and b).

12. The cosmetic composition according to claim 11 in the form of hair shampoos, shower gels, soaps, syndets, washing pastes, washing lotions, scrub preparations, foam baths, oil baths, shower baths, shaving foams, shaving lotions and shaving creams.

13. A detergent or cleaner comprising at least one compound of the general formula (I), as defined in any one of claims 1 to 8.

14. The use of at least one compound of the general formula (I) as defined in any one of claims 1 to 8 as surface-active substances.

15. The use of at least one compound of the general formula (I) as defined in any one of claims 1 to 8 as anionic surfactant for cosmetic compositions, as anionic surfactant for pharmaceutical compositions, as anionic surfactant for detergents and cleaners.

## Revendications

1. Composés de formule générale (I) 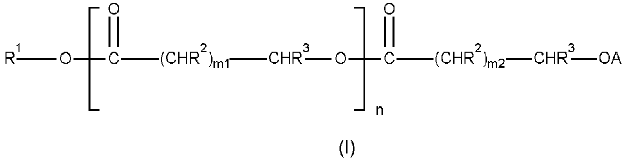 dans laquelle
R¹ représente hydrogène ou un radical hydrocarboné aliphatique, linéaire ou ramifié comprenant 1 à 30 atomes de carbone et 0, 1, 2 ou 3 doubles liaisons,
les radicaux R² sont choisis, indépendamment l'un de l'autre, parmi hydrogène, méthyle, éthyle, -OA, -COOR⁴, -CH₂-OA et -CH₂-COOR⁴, les radicaux R⁴ représentant hydrogène ou un radical hydrocarboné aliphatique, linéaire ou ramifié, comprenant 1 à 30 atomes de carbone et 0, 1, 2 ou 3 doubles liaisons,
les radicaux R³ sont choisis, indépendamment l'un de l'autre, parmi hydrogène, méthyle, éthyle, -OA, -COOR⁵, -CH₂-OA et -CH₂-COOR⁵, les radicaux R⁵ représentant hydrogène ou un radical hydrocarboné aliphatique, linéaire ou ramifié, comprenant 1 à 30 atomes de carbone et 0, 1, 2 ou 3 doubles liaisons,
A représente H ou -SO₃B, B représentant hydrogène ou un équivalent cationique,
n vaut en moyenne au moins 0,1,
m1 et m2 représentent, indépendamment l'un de l'autre, 0 ou 1,
à condition qu'au moins un des radicaux A représente -SO₃B et à condition qu'au moins un des radicaux R¹, R⁴ ou R⁵ représente un radical hydrocarboné aliphatique, linéaire ou ramifié comprenant 1 à 30 atomes de carbone et 0, 1, 2 ou 3 doubles liaisons.

2. Composés (I) selon la revendication 1, m1 et m2 ayant la même signification.

3. Composés (I) selon l'une quelconque des revendications précédentes, qui sont dérivés de l'acide lactique, de l'acide glycolique, de l'acide malique, de l'acide tartrique ou de leurs mélanges.

4. Composés (I) selon l'une quelconque des revendications précédentes, qui sont choisis parmi les composés de formule (1.1) 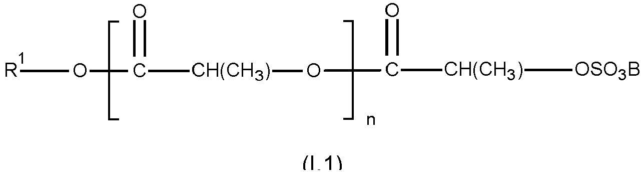 où
R¹ représente hydrogène ou un radical hydrocarboné linéaire ou ramifié comprenant 6 à 30 atomes de carbone et 0, 1, 2 ou 3 doubles liaisons,
B représente H ou un équivalent cationique et
n vaut en moyenne au moins 0,1.

5. Composés (I) selon l'une quelconque des revendications précédentes, n présentant une valeur de 0,1 à 100, de préférence de 0,15 à 50, en particulier de 0,2 à 20.

6. Composés (I) selon l'une quelconque des revendications précédentes, au moins un des radicaux R¹, R⁴ et R⁵ représentant méthyle, éthyle, n-propyle, isopropyle, n-butyle, isobutyle, sec-butyle, tert-butyle, n-pentyle, isoamyle, n-hexyle, 2-éthylhexyle, n-heptyle, n-octyle, n-nonyle, n-décyle, 2-propylheptyle, n-undécyle, n-dodécyle, n-tridécyle, n-tétradécyle, n-pentadécyle, n-hexadécyle, n-heptadécyle, n-octadécyle, n-nonadécyle, arachinyle, béhényle, lignocérinyle, mélissinyle, isotridécyle, isostéaryle, oléyle, linoléyle, linolényle ou une combinaison d'au moins deux de ces radicaux.

7. Composés (I) selon l'une quelconque des revendications précédentes, au moins un des radicaux R¹, R⁴ et R⁵ étant dérivé d'alcools linéaires saturés comprenant 8 à 18 atomes de carbone.

8. Composés (I) selon l'une quelconque des revendications précédentes, au moins un des radicaux R¹, R⁴ et R⁵ étant dérivé d'un mélange d'alcools linéaires saturés en C₁₂/C₁₄.

9. Procédé pour la préparation de composés de formule générale (I), dans lequel
a) on transforme au moins un acide hydroxycarboxylique de formule générale (I.A) 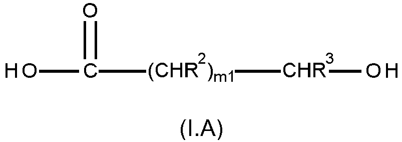 dans laquelle R², R³ et m1 sont définis comme dans l'une quelconque des revendications 1 à 8, dans une réaction d'estérification, l'estérification ayant lieu en présence d'au moins un alcool R¹-OH, R¹ étant défini comme dans l'une quelconque des revendications 1 à 8 ou le produit de l'estérification de l'acide/des acides hydroxycarboxylique(s) (I.A) étant ensuite transformé avec au moins un alcool R¹-OH,
b) on transforme le produit de réaction de l'étape a) avec un agent de sulfatation, et
c) on neutralise le cas échéant au moins partiellement le produit de réaction de l'étape b) avec une base.

10. Procédé selon la revendication 9, l'agent de sulfatation utilisé dans l'étape b) comprenant du SO₃ ou étant constitué par du SO₃.

11. Composition cosmétique et/ou pharmaceutique, contenant
a) au moins un composé de formule générale (I) tel que défini dans l'une quelconque des revendications 1 à 8,
b) au moins une substance active cosmétique ou pharmaceutique et
c) le cas échéant au moins un adjuvant cosmétique ou pharmaceutique différent des composants a) et b).

12. Composition cosmétique selon la revendication 11 sous forme de shampooings, de gels douche, de savons, de syndets, de pâtes de lavage, de lotions de lavage, de préparations d'exfoliation, de bains mousses, de bains d'huile, de bains douche, de mousses de rasage, de lotions de rasage et de crèmes de rasage.

13. Agent de lavage ou de nettoyage, contenant au moins un composé de formule générale (I) tel que défini dans l'une quelconque des revendications 1 à 8.

14. Utilisation d'au moins un composé de formule générale (I) tel que défini dans l'une quelconque des revendications 1 à 8, comme substance tensioactive.

15. Utilisation d'au moins un composé de formule générale (I) tel que défini dans l'une quelconque des revendications 1 à 8, comme tensioactif anionique pour des agents cosmétiques, comme tensioactif anionique pour des agents pharmaceutiques, comme tensioactif anionique pour des agents de lavage et de nettoyage.
